# EUROPÄISCHE PATENTSCHRIFT

(11) **EP 2 809 310 B1**
(45) Veröffentlichungstag und Bekanntmachung des Hinweises auf die Patenterteilung: **20.01.2016**
(21) Anmeldenummer: 12703448.6
(22) Anmeldetag: 02.02.2012
(51) Int. Cl.: C07D 311/62, A61K 36/15, B01D 9/00, B01D 11/02

(54) **VERFAHREN ZUR HERSTELLUNG VON TAXIFOLIN AUS HOLZ**
PROCESS FOR THE PRODUCTION OF TAXIFOLIN FROM WOOD
PROCEDE DE PRODUCTION DU TAXIFOLIN A PARTIR DE BOIS

(43) Veröffentlichungstag der Anmeldung: 10.12.2014
(73) Patentinhaber: LDA AG, 8832 Wollerau (CH)
(72) Erfinder: WIESBECK, Franz, CH-8835 Feusisberg (CH)
(74) Vertreter: Hoefer & Partner Patentanwälte mbB
(86) Internationale Anmeldenummer: PCT/EP2012/000474
(87) Internationale Veröffentlichungsnummer: WO 2013/113329

(56) Entgegenhaltungen:
- EP-A1- 2 143 435
- WO-A1-2011/155829

## Beschreibung

Die vorliegende Erfindung betrifft ein Verfahren zur Herstellung bzw. Isolation von Taxifolin aus Holz, insbesondere aus Lärchenholz.

Der Stand der Technik kennt unterschiedliche Verfahren zur Herstellung von Taxifolin (auch: Dihydroquercetin) aus Lärchenholz. Allerdings zeigen die vorbekannten Verfahren lediglich Methoden zur Herstellung kleiner Mengen, insbesondere für die Pharmaindustrie. Diese Methoden eignen sich nicht zur wirtschaftlich sinnvollen Herstellung großer Mengen dieses Wertstoffes.

Aus der EP 2143435 A1 ist ein Verfahren zur Extraktion von Taxifolin aus Holz bekannt, bei dem Holzpartikel mit wässrigen Aceton extrahiert werden und anschließend wird das Aceton-Extrakt mit Wasser versetzt, wonach die Harz- bzw. Ölphase abgetrennt wird und die Wasserphase wiederum mit Ethylacetat extrahiert wird, wonach das erhaltene Extrakt getrocknet wird. Mit diesem Verfahren werden zwei Extraktionsstufen benötigt.

Es ist Aufgabe vorliegender Erfindung, ein Verfahren anzugeben, das bei kostengünstiger und umweltverträglicher Durchführung das wirtschaftliche Herstellen von möglichst reinem Taxifolin aus Holz ermöglicht.

Die Lösung dieser Aufgabe erfolgt durch die Merkmale des Anspruchs 1. Die abhängigen Ansprüche haben bevorzugte Weiterbildungen der Erfindung zum Gegenstand.

Somit wird die Aufgabe gelöst durch ein Verfahren zur Herstellung von Taxifolin. Das Verfahren umfasst die folgenden Schritte in gegebener Reihenfolge: Zunächst erfolgt ein Aufschichten von Holzpartikeln in einem Perkolator. Bevorzugt wird hier Lärchenholz verwendet. Besonders bevorzugt werden Holzpartikel des unteren Stammbereichs und/oder des Wurzelstocks der Lärche, insbesondere der sibirischen Lärche (larix sibirica/larix dahurica), verwendet. Ursächlich hierfür ist der hohe Gehalt an Taxifolin in dem Stammabschnitt und/oder dem Wurzelstock der Lärche. Insbesondere werden Rindholz, Splintholz und Astholz entfernt und nicht extrahiert. Nach dem Aufschichten der Holzpartikel im Perkolator erfolgt ein Extrahieren der Holzpartikel mit Ethanol, zum Lösen von Harzen und Ölen und somit auch von Taxifolin. Nach dem Extrahieren erfolgt ein Abführen des ethanolischen Extraktes aus dem Perkolator. Im Anschluss erfolgt ein Aufreinigen des ethanolischen Extraktes zur Herstellung von Taxifolin mit den folgenden Schritten in gegebener Reihenfolge: (i) Erzeugen eines Gemisches aus Wasser und dem ethanolischen Extrakt; vorzugsweise wird zumindest die gleiche Menge Wasser zugemischt, wie ethanolischer Extrakt vorhanden ist, (ii) Durchmischen des Gemisches bei 70°C bis 99°C, sodass Taxifolin in der Wasserphase des Gemisches in Lösung geht; das Taxifolin geht insbesondere bei diesem Temperaturbereich aus den gelösten Harzen in das Wasser in Lösung, (iii) Abkühlen des Gemisches auf unter 65°C zum Abscheiden und Abführen der Harz-Phase und der Öl-Phase von der Wasserphase, (iv) Zugabe von Taxifolinimpfkristallen zu der Wasserphase, (v) Temperieren der Wasserphase auf 0°C bis 30°C zur Auskristallisierung des Taxifolins, und (vi) Separieren des auskristallisierten Taxifolins von der Mutterlauge.

Bevorzugt wird zum Extrahieren Ethanol mit einer Konzentration von mindestens 70%, vorzugsweise mindestens 80%, besonders vorzugsweise mindestens 95%, verwendet. Zum Lösen eines verbliebenen Restextraktes erfolgt bevorzugt ein Anlegen eines Unterdrucks am Perkolator und ein Nachspülen der Holzpartikel im Perkolator mit frischem Ethanol sowie eine anschließende Vereinigung der ethanolischen Extrakte. Vor dem Aufreinigen wird der ethanolischen Extrakte durch Evaporation, vorzugsweise auf eine Konzentration zwischen 50 und 250 g/L (Gramm pro Liter) Taxifolin, eingedampft. Die Temperatur- und Druckverhältnisse bei der Evaporation werden so gewählt, dass der Ethanolgehalt im ethanolischen Extrakt reduziert wird. Vorzugsweise erfolgt die Evaporation bei einer Temperatur zwischen 55°C und 70°C und reduziertem Druck.

Der anschließende Aufreinigungsprozess weist bevorzugte folgende weitere Schritte auf:
Das Erzeugen des Gemisches aus Wasser und dem ethanolischen Extrakt erfolg bevorzugt bei einer Temperatur zwischen 70°C und 95°C. Dadurch ist das Gemisch gut vortemperiert für die Durchmischung. Direkt vor und/oder während dem Durchmischen des Gemisches erfolgt bevorzugt eine Reduktion des Ethanolgehaltes im Gemisch durch Evaporation.

Vorteilhafterweise erfolgt das Durchmischen des Gemisches aktiv, vorzugsweise mittels eines Rührwerkes. Für ein optimales Lösen des Taxifolins in der wässrigen Phase zeigte sich ein Temperaturbereich beim Durchmischen zwischen 70°C und 99°C, vorzugsweise zwischen 70°C und 95°C, besonders vorzugsweise zwischen 80°C und 95°C, als vorteilhaft. Direkt vor und/oder während dem Durchmischen des Gemisches wird bevorzugt weiteres Wasser dem Gemisch zugefügt. Insbesondere wird zumindest die gleiche Menge Wasser zugemischt, wie Gemisch nach der Evaporation des Gemisches noch vorhanden ist.

Nach dem Durchmischen folg das Abscheiden der Harz-Phase (untere Phase) von der Wasserphase (obere Phase) und der Ölphase (oberste Phase) durch Abkühlen des Gemisches auf unter 65°C. Vorzugsweise wird das Gemisch über zumindest 30 Minuten von den 70°C bis 99°C beim Durchmischen auf 35°C bis 64°C abgekühlt. Nach dem Abkühlen erfolgt ein Separieren der Harzphase aus dem Gemisch und bevorzugt zumindest ein einmaliges Rückführen der Harz-Phase in das Gemisch vor dem Abkühlen. Vorzugsweise wird die Harz-Phase vor und/oder während dem oben genannten Durchmischen in das Gemisch zurückgeführt. Nach zumindest einmaligem Rückführen der Harze erfolgt ein Abführen der Harz-Phase und ein Separieren und Abführen der Ölphase von der Wasserphase.

Im nächsten Schritt erfolgen die Zugabe von Taxifolinimpfkristallen zu der Wasserphase und ein Abkühlen zur Auskristallisierung des Taxifolins. Daraufhin folgt ein Separieren des auskristallisierten Taxifolins von der Mutterlauge und Abführen der Mutterlauge.

Bevorzugt erfolg dann zumindest eine erneute Kristallisation mit den folgenden Schritten in gegebener Reihenfolge: Lösen des von der Mutterlauge separierten, auskristallisierten Taxifolins in Wasser bei 70°C bis 99°C, Zugabe von Taxifolinimpfkristallen, Temperieren der Wassers auf 0°C bis 30°C zur Auskristallisierung des Taxifolins, Separieren des erneut auskristallisierten Taxifolins von der Mutterlauge, und Abführen der Mutterlauge.

Bevorzugt ist vorgesehen, dass beim erstmaligen und/oder dem erneuten Kristallisieren die Zugabe der Taxifolinimpfkristalle bei einer ersten Temperatur des Wassers zwischen 30°C und 65°C, vorzugsweise zwischen 45°C und 60°C, erfolgt, und das Wasser zum Auskristallisieren auf eine zweite Temperatur zwischen 0°C und 30°C, vorzugsweise zwischen 20°C und 10°C, temperiert wird. Das Abkühlen von der ersten Temperatur auf die zweite Temperatur erfolg vorzugsweise über 5 bis 15 Stunden. Besonders vorteilhaft für die Kristallisation ist ein Halten der zweiten Temperatur über 0,5 bis 2 Stunden.

Nach dem Kristallisieren erfolgen ein Waschen des auskristallisierten Taxifolins mit kaltem Wasser (unter 50°C) und ein Trocknen des gewaschenen Taxifolins.

Im gesamten beschriebenen Verfahren, bei der Extraktion und bei der Aufreinigung, kommt bevorzugt ausschließlich Wasser und Ethanol zum Einsatz. Bevorzugt wird weder das Holz noch der Extrakt oder das Taxifolin mit anderen Stoffen als Wasser oder Ethanol behandelt. Für das Gemisch und die Kristallisation wird bevorzugt ausschließlich Wasser, ohne weitere Zusätze, verwendet. Dadurch wird jedwede Verunreinigung des Taxifolins vermieden.

Nachfolgend wird ein Ausführungsbeispiel des erfindungsgemäßen Verfahrens unter Bezugnahme auf die begleitende Zeichnung erläutert. Dabei zeigen:
- Fig. 1: einen Ablauf der Extraktion gemäß dem erfindungsgemäßen Verfahren, und
- Fig. 2: einen Ablauf der Aufreinigung von Taxifolin gemäß dem erfindungsgemäßen Verfahren.

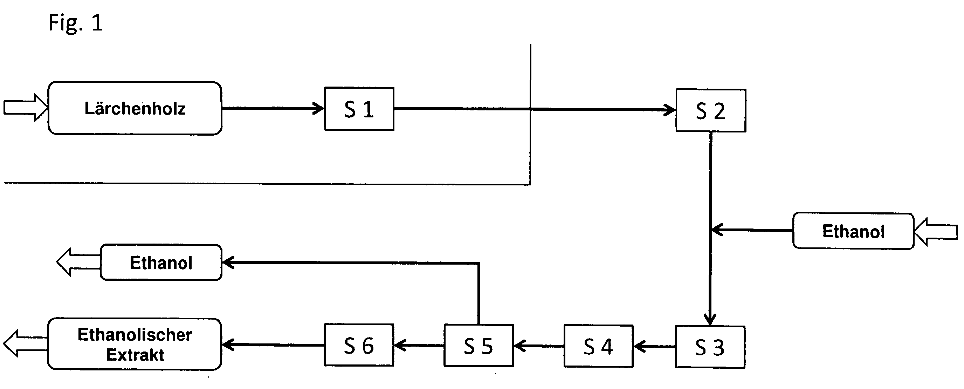

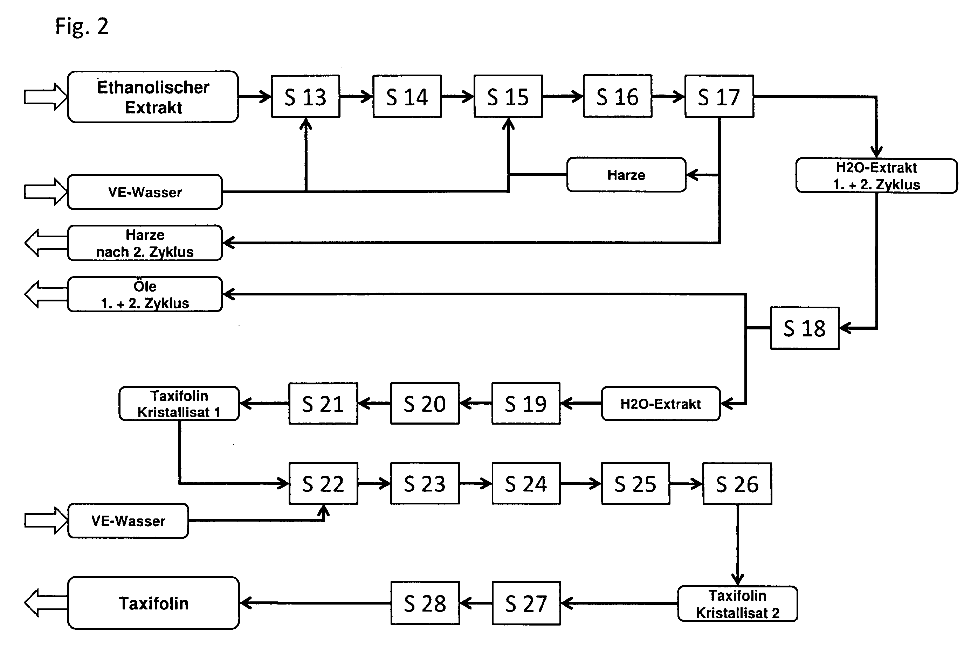

Fig. 1 zeigt die Schritte S1 bis S6 zur Extraktion von Lärchenholz. Nach dem Schritt S6 folgen die Schritte S13 bis S28 gemäß Fig. 2 zur Aufreinigung des Taxifolins.

Als Ausgangsstoff wird der untere Stammabschnitt der Lärche, insbesondere der sibirischen Lärche (*Larix sibirica*), insbesondere der dahurischen Lärche (*Larix dahurica*), insbesondere *Larix spp.* verwendet. Im Schritt S1 erfolgt ein Zerkleinern des Holzes. Dies erfolgt insbesondere mittels eines Hackers und/oder einer Hammermühle. Im Schritt S2 erfolgt ein Aufschichten der zerkleinerten Holzpartikel in einem Perkolator. Im Schritt S3 wird Ethanol in den Perkolator eingeleitet. Insbesondere wird das Ethanol oben in den Perkolator eingeleitet und unten gesammelt. Über eine Pumpe wird das Ethanol mehrmals durch den Perkolator gepumpt. Im Schritt S4 wird der gewonnene ethanolische Extrakt aus dem Perkolator abgeführt und es erfolgt im Schritt S5 eine Evaporation des ethanolischen Extraktes. Dabei wird ein Teil des Ethanols verdampft. Im Schritt S6 erfolgt ein Transfer des aufkonzentrierten, ethanolischen Extraktes zum Schritt S13 (siehe Fig. 2).

Zu Beginn der Aufreinigung des ethanolischen Extraktes zur Gewinnung von Taxifolin erfolgt ein Wechsel des Lösungsmittels. Im Schritt S13 erfolgt ein Mischen des ethanolischen Extraktes mit Wasser und eine erneute Evaporation im Schritt S14. Die Temperatur- und Druckverhältnisse im Schritt S14 sind so gewählt, dass in erster Linie Ethanol verdampft. Im Schritt S15 wird erneut Wasser zugeführt und es erfolgt ein aktives Mischen, vorzugsweise mit einem Rührwerk, so dass das Taxifolin aus den Harzen in das Wasser in Lösung geht. Hierbei wird auch von einer wässrigen Harzextraktion oder einer Flüssig/Flüssig-Extraktion im Schritt S15 gesprochen. Das Mischen im Schritt S15 erfolgt bevorzugt bei der gleichen oder höherer Temperatur, als die Evaporation in S14.

Im Schritt S16 erfolgt ein Abkühlen des Gemisches aus S15. Insbesondere wird über eine längere Zeit abgekühlt, so dass sich die Harzphase in dem Gemisch absetzt. Durch dieses Abkühlen trennt sich das Gemisch in eine Harzphase (untere Phase) und in eine flüssige Phase (obere Phase). In dieser oberen Phase befinden sich ganz oben Öle und zwischen der Harzphase und den Ölen befindet sich eine wässrige Phase. Im Schritt S17 werden die Öle und die wässrige Phase von dem Harz separiert. Das Harz wird nochmals zurückgeführt zum Schritt S15. Dieses Zurückführen der Harze wird zumindest einmalig durchgeführt. Nach dem zweiten Zyklus werden die Harze abgeführt. Die obere Phase, bestehend aus Ölen und der wässrigen Phase aus beiden Zyklen, wird im Schritt S18 separiert. Dabei werden die Öle abgeführt. Das verbleibende wässrige Extrakt wird in den folgenden schritten durch Kristallisationsprozesse und Waschen weiterverarbeitet.

Im Schritt S19 erfolgt eine Zugabe von Taxifolinimpfkristallen zu der wässrigen Phase. Im Schritt S20 wird langsam abgekühlt, so dass das Taxifolin auskristallisiert. Die Mutterlauge wird im Schritt S21 abgeführt und das auskristallisierte Taxifolin in Schritt S22 erneut mit Wasser vermischt. Dabei wird eine erhöhte Temperatur gewählt, so dass das Taxifolin erneut im Wasser in Lösung geht. Im Schritt S23 erfolgt ein Abkühlen und im Schritt S24 eine Zugabe von Taxifolinimpfkristallen. Im Schritt S25 wird weiter langsam abgekühlt, so dass das Taxifolin auskristallisiert. Im Schritt S26 wird wiederum die Mutterlauge abgeführt und das auskristallisierte Taxifolin kann im Schritt S27 gewaschen werden. Das Waschen erfolgt unter Zugabe von kaltem Wasser. Anschließend erfolgt eine Trocknung im Schritt S28, bevorzugt unter Schutzgas. Nach dem Trocknen wird ein Taxifolingehalt von 85-95 %erreicht.

Der Taxifolingehalt im Endprodukt wurde basierend auf dem bekannten Molekulargewicht von Taxifolin und der Peakfläche der etablierten flüssigchromatografischen Analysemethode (HPLC/UPLC) bestimmt. Die bestimmten Taxifolingehalte lagen hierbei zwischen 90-92%. Die Reinheit der Proben wurde ebenfalls Flüssigkeitschromatografisch über den Peakflächenanteil (% area) des Probenchromatograms bestimmt und lag im Schnitt bei 82 - 83 % area. Die Hauptverunreinigungen setzt sich aus 6 % area Dihydrokaempferol, 7 % area Quercetin 0.8 % area Naringenin, 0.6 % area Epitaxifolin zusammen.

## Patentansprüche

1. Verfahren zur Herstellung von Taxifolin, umfassend die folgenden Schritte:
Aufschichten von Holzpartikeln in einem Perkolator, Extrahieren der Holzpartikel im Perkolator mit Ethanol zum Lösen von zumindest Harzen, Ölen und Taxifolin, Abführen des ethanolischen Extraktes aus dem Perkolator, und Aufreinigen des ethanolischen Extraktes zur Herstellung von Taxifolin mit den folgenden Schritten in gegebener Reihenfolge:
- Erzeugen eines Gemisches aus Wasser und dem ethanolischen Extrakt,
- Durchmischen des Gemisches bei 70°C bis 99°C, sodass Taxifolin in der Wasserphase des Gemisches in Lösung geht,
- Abkühlen des Gemisches auf unter 65°C zum Abscheiden und Abführen der Harz-Phase und der Öl-Phase von der Wasserphase,
- Zugabe von Taxifolinimpfkristallen zu der Wasserphase,
- Temperieren der Wasserphase auf 0°C bis 30°C zur Auskristallisierung des Taxifolins, und
- Separieren des auskristallisierten Taxifolins von der Mutterlauge.

2. Verfahren nach Anspruch 1, **gekennzeichnet durch** Aufkonzentrieren des ethanolischen Extraktes **durch** Evaporation vor der Aufreinigung.

3. Verfahren nach einem der vorhergehenden Ansprüche, **dadurch gekennzeichnet, dass** zum Extrahieren Ethanol mit einer Konzentration von mindestens 70%, vorzugsweise mindestens 80%, besonders vorzugsweise mindestens 95%, verwendet wird.

4. Verfahren nach einem der vorhergehenden Ansprüche, **dadurch gekennzeichnet, dass** das Erzeugen des Gemisches bei einer Temperatur zwischen 70°C und 95°C erfolgt.

5. Verfahren nach einem der vorhergehenden Ansprüche, **gekennzeichnet durch** eine Reduktion des Ethanolgehaltes im Gemisch **durch** Evaporation direkt vor und/oder während dem Durchmischen des Gemisches.

6. Verfahren nach einem der vorhergehenden Ansprüche, **dadurch gekennzeichnet, dass** das Durchmischen des Gemisches aktiv, vorzugsweise mittels eines Rührwerkes, erfolgt.

7. Verfahren nach einem der vorhergehenden Ansprüche, **dadurch gekennzeichnet, dass** direkt vor und/oder während dem Durchmischen des Gemisches weiteres Wasser dem Gemisch zugefügt wird.

8. Verfahren nach einem der vorhergehenden Ansprüche, **dadurch gekennzeichnet, dass** das Durchmischen des Gemisches bei 80°C bis 95°C erfolgt.

9. Verfahren nach einem der vorhergehenden Ansprüche, **gekennzeichnet durch** zumindest einmaliges Rückführen der abgeschiedene Harz-Phase in das Gemisch vor und/oder während dem Durchmischen.

10. Verfahren nach einem der vorhergehenden Ansprüche, **gekennzeichnet durch** zumindest eine erneute Kristallisation mit den folgenden Schritten in gegebener Reihenfolge: Lösen des von der Mutterlauge separierten, auskristallisierten Taxifolins in Wasser bei 70°C bis 99°C, Zugabe von Taxifolinimpfkristallen in das Wasser, Temperieren der Wassers auf 0°C bis 30°C zur Auskristallisierung des Taxifolins, Separieren des erneut auskristallisierten Taxifolins von der Mutterlauge, und Abführen der Mutterlauge.

11. Verfahren nach einem der vorhergehenden Ansprüche, **dadurch gekennzeichnet, dass** beim erstmaligen und/oder dem erneuten Kristallisieren die Zugabe der Taxifolinimpfkristalle bei einer ersten Temperatur des Wassers zwischen 30°C und 65°C, vorzugsweise zwischen 45°C und 60°C, erfolgt, und das Wasser zum Auskristallisieren auf eine zweite Temperatur zwischen 0°C und 30°C, vorzugsweise zwischen 20°C und 10°C, temperiert wird.

12. Verfahren Anspruch 11, **dadurch gekennzeichnet, dass** das Abkühlen von der ersten Temperatur auf die zweite Temperatur über 5 bis 15 Stunden erfolgt.

13. Verfahren nach einem der vorhergehenden Ansprüche, **gekennzeichnet durch** Waschen des auskristallisierten Taxifolins mit Wasser unter 50°C und Trocknen des gewaschenen Taxifolins.

## Claims

1. Process for the production of taxifolin comprising the following steps:
stacking wood particles in a percolator, extracting the wood particles in the percolator with ethanol in order to dissolve at least resins, oils and taxifolin, removing the ethanolic extract from the percolator, and purifying the ethanolic extract in order to produce taxifolin, with the following steps in the given sequence:
- producing a mixture of water and the ethanolic extract,
- thorough mixing of the mixture at 70 ºC to 99 ºC, so that taxifolin dissolves in the aqueous phase of the mixture,
- cooling the mixture to below 65 ºC in order to separate out and remove the resin phase and the oily phase from the aqueous phase,
- adding taxifolin seed crystals to the aqueous phase,
- controlling the temperature of the aqueous phase at 0 ºC to 30 ºC in order to crystallise out the taxifolin, and
- separating the crystallised-out taxifolin from the mother liquor.

2. Process according to claim 1, **characterised in that** the ethanolic extract is concentrated by evaporation before the purification.

3. Process according to one of the preceding claims, **characterised in that** ethanol having a concentration of at least 70 %, preferably at least 80 %, particularly preferably at least 95 % is used for the extraction.

4. Process according to one of the preceding claims, **characterised in that** the mixture is produced at a temperature of between 70 ºC. and 95 ºC.

5. Process according to one of the preceding claims, **characterised in that** the ethanol content in the mixture is reduced by evaporation directly before and/or during the thorough mixing of the mixture.

6. Process according to one of the preceding claims, **characterised in that** the thorough mixing of the mixture is carried out actively, preferably by means of a stirrer.

7. Process according to one of the preceding claims, **characterised in that** further water is added to the mixture directly before and/or during the thorough mixing of the mixture.

8. Process according to one of the preceding claims, **characterised in that** the thorough mixing of the mixture is carried out at 80 ºC to 95 ºC.

9. Process according to one of the preceding claims, **characterised in that** the resin phase separated out is recycled at least once into the mixture before and/or during the thorough mixing.

10. Process according to one of the preceding claims, **characterised in that** at least one renewed crystallisation is carried out with the following steps in the given sequence: dissolving the crystallised-out taxifolin which has been separated from the mother liquor in water at 70 ºC to 99 ºC, adding taxifolin seed crystals into the water, controlling the temperature of the water at 0 ºC to 30 ºC in order to crystallise out the taxifolin, separating the renewed crystallised-out taxifolin from the mother liquor, and removing the mother liquor.

11. Process according to one of the preceding claims, **characterised in that** during the first and/or the renewed crystallisation, the addition of the taxifolin seed crystals is carried out at a first temperature of the water of between 30 ºC and 65 ºC, preferably between 45 ºC and 60 ºC, and the temperature of the water for the crystallising out is controlled at a second temperature of between 0 ºC and 30 ºC, preferably between 20 ºC and 10 ºC.

12. Process according to claim 11, **characterised in that** the cooling from the first temperature to the second temperature is carried out over 5 to 15 hours.

13. Process according to one of the preceding claims, **characterised in that** the crystallised-out taxifolin is washed with water at below 50 ºC and the washed taxifolin is dried.

## Revendications

1. Procédé de production de taxifoline comprenant les étapes suivantes :
empilement de particules de bois dans un percolateur, extraction des particules de bois dans le percolateur avec de l'éthanol pour dissoudre au moins des résines, des huiles et de la taxifoline, évacuation de l'extrait d'éthanol du percolateur et purification de l'extrait d'éthanol pour la production de taxifoline avec les étapes suivantes selon l'ordre donné :
- préparation d'un mélange composé d'eau et de l'extrait d'éthanol,
- mélange du mélange à 70 °C à 99 °C, de sorte que la taxifoline en phase aqueuse du mélange entre en solution,
- refroidissement du mélange à moins de 65 °C pour séparer et évacuer la phase résineuse et la phase huileuse de la phase aqueuse,
- ajout de germes cristallins de taxifoline à la phase aqueuse,
- modération de la température de la phase aqueuse à 0 °C à 30 °C pour cristalliser la taxifoline et
- séparation de la taxifoline cristallisée de la liqueur mère.

2. Procédé selon la revendication 1, **caractérisé par** la concentration de l'extrait d'éthanol par évaporation avant la purification.

3. Procédé selon l'une quelconque des revendications précédentes, **caractérisé en ce que** pour l'extraction on utilise de l'éthanol présentant une concentration d'au moins 70 %, de préférence d'au moins 80 %, de manière particulièrement préférée d'au moins 95 %.

4. Procédé selon l'une quelconque des revendications précédentes, **caractérisé en ce que** la préparation du mélange s'effectue à une température entre 70 °C et 95 °C.

5. Procédé selon l'une quelconque des revendications précédentes, **caractérisé par** une réduction de la teneur en éthanol dans le mélange par évaporation directement avant et/ou pendant le mélange du mélange.

6. Procédé selon l'une quelconque des revendications précédentes, **caractérisé en ce que** le mélange du mélange s'effectue de manière active, de préférence au moyen d'un mélangeur.

7. Procédé selon l'une quelconque des revendications précédentes, **caractérisé en ce que** directement avant et/ou pendant le mélange du mélange de l'eau supplémentaire est rajoutée au mélange.

8. Procédé selon l'une quelconque des revendications précédentes, **caractérisé en ce que** le mélange du mélange s'effectue à 80 °C à 95 °C.

9. Procédé selon l'une quelconque des revendications précédentes, **caractérisé par** le recyclage au moins une fois de la phase résineuse séparée dans le mélange avant et/ou pendant le mélange.

10. Procédé selon l'une quelconque des revendications précédentes, **caractérisé par** au moins une nouvelle cristallisation avec les étapes suivantes selon l'ordre donné : dissolution de la taxifoline cristallisée, séparée de la liqueur mère dans de l'eau à 70 °C à 99 °C, ajout de germes cristallins de taxifoline dans l'eau, modération de la température de l'eau à 0 °C à 30 °C pour la cristallisation de la taxifoline, séparation de la taxifoline à nouveau cristallisée de la liqueur mère et évacuation de la liqueur mère.

11. Procédé selon l'une quelconque des revendications précédentes, **caractérisé en ce que** lors de la première et/ou la nouvelle cristallisation l'ajout des germes cristallins de taxifoline s'effectue à une première température de l'eau entre 30 °C et 65 °C, de préférence entre 45 °C et 60 °C, et l'eau pour la cristallisation est tempérée à une deuxième température entre 0 °C et 30 °C, de préférence entre 20 °C et 10 °C.

12. Procédé selon la revendication 11, **caractérisé en ce que** le refroidissement de la première température à la deuxième température s'effectue sur 5 à 15 heures.

13. Procédé selon l'une quelconque des revendications précédentes, **caractérisé par** le lavage de la taxifoline cristallisée avec de l'eau à moins de 50 °C et le séchage de la taxifoline lavée.
